# EUROPÄISCHE PATENTANMELDUNG

(11) **EP 4 397 363 A1**
(43) Veröffentlichungstag der Anmeldung: **10.07.2024**
(21) Anmeldenummer: 23152275.6
(22) Anmeldetag: 18.01.2023
(51) Int. Cl.: A61N 5/06, A61H 33/06

(54) **INFRAROTSTRAHLER**

(30) Priorität: 09.01.2023 EP 23150708
(71) Anmelder: Mattheiss, Gerd, 7203 Trimmis (CH)
(72) Erfinder: PECHER, Otto, 85653 Aying b. München (DE); ZEIGER, Thomas, 6134 Vomp (AT); MATTHEISS, Gerd, 7203 Trimmis (CH)
(74) Vertreter: Fuchs Patentanwälte Partnerschaft mbB

(57) **Zusammenfassung**

Eine Infrarotstrahler-Anordnung (100) für ein Wärmebehandlungssystem (400) umfasst wenigstens eine Wärmequelle (150, 152), wenigstens einen Reflektor (140, 142, 144), der dazu ausgebildet ist, Infrarotstrahlung, die mittels der wenigstens einen Wärmequelle (150, 152) erzeugt ist, in Richtung auf einen Benutzer des Wärmebehandlungssystems (400) umzulenken, und wenigstens ein Gestell (105), an dem die wenigstens eine Wärmequelle (150, 152) und der wenigstens eine Reflektor (140, 142, 144) angebracht sind. Während einer vorgesehenen Verwendung des Wärmebehandlungssystems (400) ist der wenigstens eine Reflektor (140, 142, 144) mechanischer und/oder chemischer Wechselwirkung mit einer Umgebung des Wärmebehandlungssystems (400) ausgesetzt ist. Der wenigstens eine Reflektor (140, 142, 144) ist zudem reversibel lösbar an dem wenigstens einen Gestell (105) angebracht.

## Beschreibung

Die Anmeldung betrifft das Gebiet der Wärmebehandlungssysteme. Die Anmeldung betrifft insbesondere eine Infrarotstrahler-Anordnung für ein Wärmebehandlungssystem, ein Wärmebehandlungssystem und eine Wärmekabine umfassend ein solches Wärmebehandlungssystem.

### Technischer Hintergrund

Wärmebehandlungssysteme sind bekannt. Sie werden üblicherweise dazu verwendet, Wärme in Form von Wärme- bzw. Infrarotstrahlung auf den Körper oder bestimmte Körperregionen eines Benutzers aufzubringen. Eine Wärmebehandlung geschieht oft als therapeutische oder therapiebegleitende Maßnahme, etwa im Rahmen physiotherapeutischer Behandlungen, oder allgemein zur Förderung des Wohlbefindens oder der Erholung.

Es besteht ein fortwährender Bedarf nach verbesserten Techniken zur Wärmebehandlung.

### Abriss der Erfindung

Diese Aufgabe wird durch eine Infrarotstrahler-Anordnung für ein Wärmebehandlungssystem gemäß Anspruch 1, ein Wärmebehandlungssystem gemäß Anspruch 10 und eine Wärmekabine gemäß Anspruch 15 gelöst.

Demnach wird gemäß einem ersten Aspekt eine Infrarotstrahler-Anordnung für ein Wärmebehandlungssystem vorgestellt. Die Infrarotstrahler-Anordnung umfasst wenigstens eine Wärmequelle, wenigstens einen Reflektor, der dazu ausgebildet ist, Infrarotstrahlung, die mittels der wenigstens einen Wärmequelle erzeugt ist, in Richtung auf einen Benutzer des Wärmebehandlungssystems umzulenken, und wenigstens ein Gestell, an dem die wenigstens eine Wärmequelle und der wenigstens eine Reflektor angebracht sind. Während einer vorgesehenen Verwendung des Wärmebehandlungssystems ist der wenigstens eine Reflektor mechanischer und/oder chemischer Wechselwirkung mit einer Umgebung des Wärmebehandlungssystems ausgesetzt. Der wenigstens eine Reflektor ist zudem reversibel lösbar an dem wenigstens einen Gestell angebracht.

Eine solche Infrarotstrahler-Anordnung begünstigt durch die Verwendung umgelenkter Infrarotstrahlung eine gleichmäßige Wärmeverteilung auch über einen größeren Behandlungsbereich, etwa im Gegensatz zur Verwendung direkter Infrarotstrahlung. Eine solche Infrarotstrahler-Anordnung begünstigt außerdem eine effiziente Nutzung erzeugter Wärmestrahlung. Insbesondere gestattet die Verwendung umgelenkter Infrarotstrahlung eine wenigstens teilweise verborgene Anordnung der Wärmequellen, wodurch eine Schutzabdeckung der Wärmequellen im Strahlungsweg zumindest teilweise vermeidbar ist. Außerdem begünstigt eine solche Infrarotstrahler-Anordnung eine verbesserte Sauberkeit und Hygiene mittels des reversibel lösbaren Reflektors. Insbesondere lässt sich durch die Verwendung umgelenkter Infrarotstrahlung ein Bereich innerhalb der Infrarotstrahler-Anordnung, der bei einer Wärmebehandlung einer Schweißabsonderung des Benutzers oder anderweitiger mechanischer und/oder chemischer Wechselwirkung mit einer Umgebung ausgesetzt ist, im Wesentlichen auf den Reflektor begrenzen. Die reversible Lösbarkeit des Reflektors erleichtert dabei ein anschließendes Austauschen des Reflektors oder einen zeitweiligen Ausbau des Reflektors, beispielsweise zum Zweck der Reinigung.

Die Infrarotstrahler-Anordnung kann so ausgebildet sein, dass der wenigstens eine Reflektor von dem wenigstens einen Gestell reversibel lösbar ist, wenn die Infrarotstrahler-Anordnung an einer Benutzeraufnahme des Wärmebehandlungssystems angebracht ist.

Das wenigstens eine Gestell kann ausgebildet sein zum Anbringen der Infrarotstrahler-Anordnung an einer Benutzeraufnahme des Wärmebehandlungssystems. Die Infrarotstrahler-Anordnung kann dabei derart an der Benutzeraufnahme anbringbar sein, dass Infrarotstrahlung, die mittels der wenigstens einen Wärmequelle erzeugt ist, in Richtung auf einen Benutzer, der mittels der Benutzeraufnahme aufgenommen ist, umgelenkt wird.

Die Infrarotstrahler-Anordnung kann wenigstens zwei Wärmequellen umfassen, die zu verschiedenen Seiten eines Mittenbereichs des wenigstens einen Reflektors angeordnet sind. Der wenigstens eine Reflektor kann dabei mehrere Reflektionsflächen umfassen, die zu verschiedenen Seiten des Mittenbereichs des Reflektors ausgebildet sind. Jeder der wenigstens zwei Wärmequellen kann jeweils wenigstens einer der Reflektionsflächen zugeordnet sein.

Die Infrarotstrahler-Anordnung kann ferner wenigstens eine Wärmesensoraufnahme umfassen, die dazu vorgesehen ist, wenigstens einen Wärmesensor des Wärmebehandlungssystems aufzunehmen. Der wenigstens eine Wärmesensor kann dazu ausgebildet sein, während der vorgesehenen Verwendung des Wärmebehandlungssystems eine Temperatur in einem Hautbereich des Benutzers zu erfassen. Der wenigstens eine Reflektor kann dabei dazu ausgebildet sein, die Infrarotstrahlung wenigstens teilweise in Richtung auf den Hautbereich des Benutzers umzulenken. Die Wärmesensoraufnahme kann dazu ausgebildet sein, den wenigstens einen Wärmesensor an einer in Bezug auf die wenigstens eine Wärmequelle abgewandten Seite des wenigstens einen Reflektors aufzunehmen.

Der wenigstens eine Reflektor kann wenigstens eine Reflektoraussparung aufweisen. Die wenigstens eine Wärmesensoraufnahme kann dazu ausgebildet sein, den wenigstens einen Wärmesensor derart aufzunehmen, dass ein Messfeld des wenigstens einen Wärmesensors sich wenigstens teilweise durch die wenigstens eine Reflektoraussparung erstreckt.

Der wenigstens eine Reflektor kann ein Reflektorblech umfassen. Zusätzlich oder alternativ kann der Reflektor Metall umfassen.

Die Infrarotstrahler-Anordnung kann ferner wenigstens eine Schweißabtropfkontur und/oder wenigstens einen Schweißauffang umfassen. Während der vorgesehenen Verwendung des Wärmebehandlungssystems kann der wenigstens eine Reflektor dabei einer Schweißabsonderung des Benutzers ausgesetzt sein.

Die wenigstens eine Schweißabtropfkontur und/oder der wenigstens eine Schweißauffang ermöglichen verbesserte Sauberkeit und Hygiene bei einer Verwendung der Infrarotstrahler-Anordnung. Die wenigstens eine Schweißabtropfkontur und/oder der wenigstens eine Schweißauffang ermöglichen zudem ein verbessertes Auffangen von Schweißabsonderungen des Benutzers während einer Wärmebehandlung, insbesondere zum Zweck einer chemischen Analyse.

Gemäß einem weiteren Aspekt wird ein Wärmebehandlungssystem vorgestellt. Das Wärmebehandlungssystem umfasst eine Infrarotstrahler-Anordnung der hier vorgestellten Art, und eine Benutzeraufnahme, an der die Infrarotstrahler-Anordnung angebracht ist und die zur sitzenden, liegenden und/oder stehenden Aufnahme des Benutzers des Wärmebehandlungssystems ausgebildet ist.

Die Benutzeraufnahme des Wärmebehandlungssystems kann ferner wenigstens eine Anliegefläche umfassen, die dem Benutzer zugewandt ist und die eine Aussparung aufweist, die mit einem zur Wärmebehandlung vorgesehenen Hautbereich des Benutzers in einer Bestrahlungsrichtung wenigstens teilweise überlappt.

Eine Größe und/oder eine Form der Aussparung kann variabel verstellbar bzw. anpassbar sein. Die Benutzeraufnahme kann wenigstens ein verstellbares Anliegeelement umfassen, das die Aussparung, insbesondere zumindest einen Bereich der Aussparung, in wenigstens einer Richtung verstellbar begrenzt.

Die Infrarotstrahler-Anordnung kann in einem von der Anliegefläche abgewandten Bereich der Benutzeraufnahme angeordnet sein derart, dass Infrarotstrahlung, die mittels der wenigstens einen Wärmequelle erzeugt ist, mittels des wenigstens einen Reflektors durch die Aussparung in Richtung auf den Benutzer umgelenkt ist.

Die Benutzeraufnahme kann eine Stützstruktur in Form eines Sitzes, einer Liege und/oder einer Lehne umfassen und zur sitzenden, liegenden und/oder stehenden Aufnahme des Benutzers vorgesehen sein. Die Anliegefläche kann dabei einer dem Benutzer zugewandten Seite der Stützstruktur entsprechen.

Die wenigstens eine Wärmequelle kann derart angeordnet sein, dass ein überwiegender Anteil der Infrarotstrahlung, die mittels der wenigstens einen Wärmequelle erzeugt ist und durch die Aussparung in Richtung auf den Benutzer tritt, umgelenkte Infrarotstrahlung ist.

Die wenigstens eine Wärmequelle kann eine steuerbare, insbesondere in Bezug auf ihre Heizleistung steuerbare, Wärmequelle sein.

Das Wärmebehandlungssystem kann dabei ferner wenigstens einen Wärmesensor umfassen, der in der Infrarotstrahler-Anordnung aufgenommen ist und der dazu ausgebildet ist, während der vorgesehenen Verwendung des Wärmebehandlungssystems eine Temperatur in einem Hautbereich des Benutzers zu erfassen und ein Sensorsignal auszugeben, das auf die erfasste Temperatur hinweist.

Das Wärmebehandlungssystem kann dabei ferner eine Steuerungseinrichtung umfassen, die dazu ausgebildet ist, das Sensorsignal zu empfangen und wenigstens teilweise auf der Grundlage des empfangenen Sensorsignals die wenigstens eine Wärmequelle in Bezug auf eine Heizleistung zu steuern.

Die wenigstens eine Wärmequelle, der wenigstens eine Reflektor, der wenigstens eine Wärmesensor und die Steuerungseinrichtung können Bestandteile einer Wärmebehandlungsvorrichtung des Wärmebehandlungssystems sein.

Gemäß einem weiteren Aspekt wird eine Wärmekabine vorgestellt. Die Wärmekabine umfasst ein Wärmebehandlungssystem der hier vorgestellten Art.

### Kurze Beschreibung der Zeichnungen

Weitere Aufgaben, Merkmale und Vorzüge der Erfindung werden anhand der Zeichnungen und der ausführlichen Beschreibung deutlich. Es zeigen:
Fig. 1 eine Schrägansicht einer Infrarotstrahler-Anordnung gemäß einem Beispiel;
Fig. 2 eine Explosionsansicht der Infrarotstrahler-Anordnung aus Fig. 1;
Fig. 3A und 3B Vorderansichten verschiedener Konfigurationen einer Benutzeraufnahme für ein Wärmebehandlungssystem gemäß einem Beispiel;
Fig. 4 eine Seitenansicht eines Wärmebehandlungssystems gemäß einem Beispiel;
Fig. 5 eine Wärmekabine gemäß einigen Beispielen, und
Fig. 6 eine Wärmebehandlungsvorrichtung gemäß einem Beispiel.

### Ausführliche Beschreibung

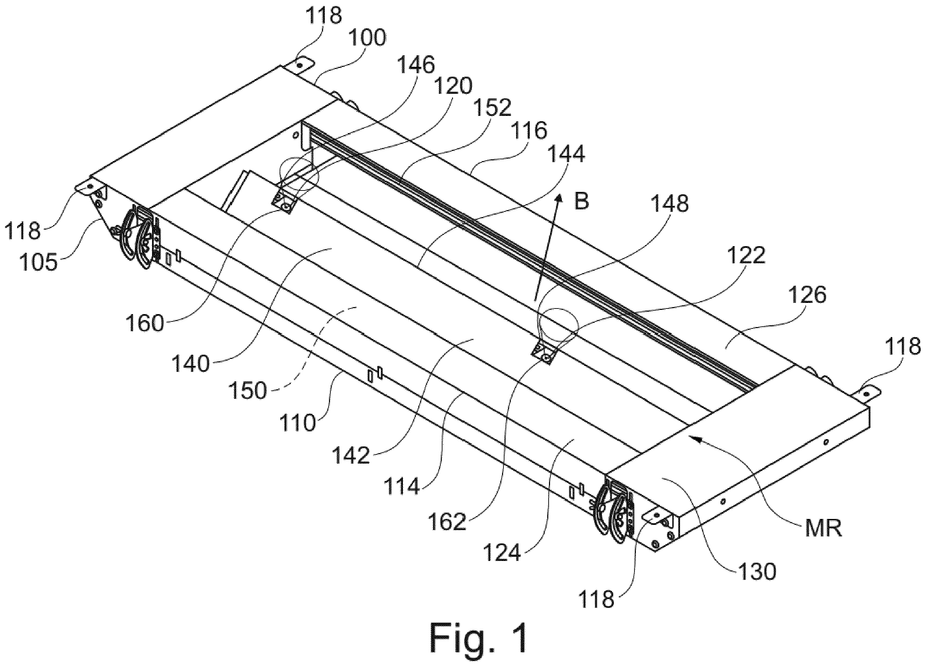

Fig. 1 zeigt schematisch und exemplarisch eine Schrägansicht einer Infrarotstrahler-Anordnung 100. Die Infrarotstrahler-Anordnung 100 ist zur Verwendung in einem Wärmebehandlungssystem und/oder einer Wärmebehandlungsvorrichtung, wie im Nachfolgenden jeweils beschrieben, vorgesehen.

Die Infrarotstrahler-Anordnung 100 umfasst ein Gestell 105, in dem mehrere Wärmequellen 150, 152 und ein Reflektor 140 angeordnet sind. Die Wärmequellen 150, 152 sind in Seitengehäusen 114, 116 des Gestells 105 untergebracht. Der Reflektor 140 ist dabei zwischen den Wärmequellen 150, 152 angeordnet. Der Reflektor 140 ist dazu vorgesehen, Infrarotstrahlung, die mittels der Wärmequellen 150,152 erzeugt wird und die so aus verschiedenen seitlichen Richtungen auf den Reflektor 140 trifft, in eine Bestrahlungsrichtung B der Infrarotstrahler-Anordnung 100 umzulenken. Um ein Austreten der umgelenkten Infrarotstrahlung aus der Infrarotstrahler-Anordnung 100 zu gestatten, weist das Gestell 105 ausgehend von dem Reflektor 140 in der Bestrahlungsrichtung B eine Öffnung 112 auf.

In dem gezeigten Beispiel erstreckt sich die Öffnung 112 zwischen den Oberseiten der Seitengehäuse 114, 116 sowie im Wesentlichen über eine gesamte Erstreckungsfläche des Reflektors 140. Der Reflektor 140 ist zudem reversibel lösbar an dem Gestell 105 angebracht, beispielsweise durch elastisches Klemmen oder Verschrauben. Die Öffnung 112 ermöglicht in dem gezeigten Beispiel ein Entnehmen des Reflektors 140 aus dem Gestell 105 sowie ein Einsetzen des Reflektors 140 in das Gestell 105 jeweils durch die Öffnung 112. Dadurch ist beispielsweise ein Austauschen und/oder Reinigen des Reflektors 140 begünstigt. In anderen Beispielen erfolgen das Entnehmen und das Einsetzen des Reflektors 140 auf anderem Weg als durch die Öffnung 112, beispielsweise durch eine Öffnung an einer Seite des Gestells 105 und/oder mittels eines reversibel entfernbaren Bodens 110 des Gestells 105.

Die Infrarotstrahler-Anordnung 100 weist in einem Randbereich des Gestells 105 mehrere Befestigungsmittel 118 auf. Die Befestigungsmittel 118 dienen dazu, die Infrarotstrahler-Anordnung 100 in einem Wärmebehandlungssystem, beispielsweise an einer Benutzeraufnahme eines Wärmebehandlungssystems, wie im Nachfolgenden beschrieben, zu befestigen.

Mittels der beschriebenen Anordnung von Wärmequellen 150, 152 und Reflektor 140 ermöglicht die Infrarotstrahler-Anordnung 100 das Aufbringen von Wärme in Form von Infrarotstrahlung auf einen Benutzer, bzw. einen Hautbereich des Benutzers, der sich in einem Bestrahlungsbereich der Infrarotstrahler-Anordnung 100 in der Bestrahlungsrichtung B befindet.

Die Anordnung von Wärmequellen 150, 152 und Reflektor 140 begünstigt dabei eine effiziente Bestrahlung unter vielfältigen Einsatzbedingungen der Infrarotstrahler-Anordnung 100 bei gleichzeitig verbesserter Sauberkeit und Hygiene. Das ist teilweise dadurch erzielt, dass eine transparente Schutzabdeckung der Öffnung 112, etwa in Form eines Deckglases, wie sie bei herkömmlichen Infrarotstrahler-Anordnungen typischerweise vorgesehen sind, vermeidbar ist. Die mittels der Wärmequellen 150, 152 erzeugte Infrarotstrahlung wird demnach auch nicht durch eine solche Schutzabdeckung teilweise absorbiert.

In Hinsicht auf eine Benutzungssicherheit ist das Vermeiden einer Schutzabdeckung dadurch ermöglicht, dass die Wärmequellen 150, 152 vor versehentlicher Berührung geschützt, beispielsweise in den Seitengehäusen 114, 116, untergebracht werden können.

In Hinsicht auf Sauberkeit und Hygiene ist eine Schutzabdeckung bei der Infrarotstrahler-Anordnung 100 dadurch vermeidbar, dass Bereiche der Infrarotstrahler-Anordnung 100, die bei einer Benutzung besonderer mechanischer und/oder chemischer Wechselwirkung mit der Umgebung, beispielweise Schweißabsonderungen des Benutzers, ausgesetzt sind, nämlich der Reflektor 140, für eine Reinigung leicht zugänglich und reversibel aus dem Gestell 105 entfernbar sind.

Die beschriebene Anordnung begünstigt so besonders einen Einsatz der Infrarotstrahler-Anordnung 100 mit aufwärts gerichteter und/oder aufwärts geneigter Bestrahlungsrichtung B. Das ist beispielsweise vorteilhaft, wenn die Infrarotstrahler-Anordnung 100 für Wärmebehandlungen im Rückenbereich eines Benutzers vorgesehen ist und zu diesem Zweck in einer geneigten und/oder neigbaren Rückenlehne einer zur sitzenden und/oder liegenden Aufnahme des Benutzers ausgebildeten Benutzeraufnahme angeordnet ist. Unter solchen und ähnlichen Einsatzbedingungen ist der Reflektor 140 üblicherweise in erhöhtem Maß Schweißabsonderungen des Benutzers ausgesetzt.

An der dem Benutzer zugewandten Seite jedes der Seitengehäuse 114, 116 ist jeweils eine Schweißabtropfkontur 124, 126 angeordnet. Die Schweißabtropfkonturen 124, 126 sind dazu vorgesehen, Schweißabsonderungen des Benutzers, die außerhalb der Öffnung 112 auf die Infrarotstrahler-Anordnung 100 treffen oder sich dort niederschlagen, durch die Öffnung 112 in Richtung auf den Reflektor 140 zu leiten. Eine Verunreinigung der Infrarotstrahler-Anordnung 100 an der Außenseite sowie einer Umgebung der Infrarotstrahler-Anordnung 100 infolge von Schweißabsonderungen lässt sich so verringern. Zudem ist dadurch ein Auffangen von Schweiß des Benutzers, beispielsweise zum Zweck einer Analyse, wie nachfolgend beschrieben, begünstigt.

In einem Bereich des Gestells 105, der bei einer vorgesehenen Montage der Infrarotstrahler-Anordnung 100 einem unteren Bereich des Gestells 105 entspricht, umfasst das Gestell 105 einen Schweißauffang 130. Der Schweißauffang 130 ist dazu vorgesehen, während einer Wärmebehandlung Schweißabsonderungen des Benutzers, die über die Schweißabtropfkonturen 124, 126 sowie direkt durch die Öffnung 112 auf den Reflektor 140 treffen und von dort ablaufen, aufzufangen. Der so aufgefangene Schweiß lässt sich hygienisch entsorgen. Nach Bedarf lässt sich der so aufgefangene Schweiß zudem chemisch analysieren, etwa um Rückschlüsse auf einen körperlichen Zustand des Benutzers zu gestatten.

Die beschriebene Anordnung von Reflektor 140 und in Bezug auf die Bestrahlungsrichtung B jeweils seitlich davon untergebrachten Wärmequellen 150, 152 ermöglicht darüber hinaus die Verwendung von hauptsächlich umgelenkter anstatt direkter Infrarotstrahlung für eine Wärmebehandlung. Eine gleichmäßige bzw. auf eine vorgesehene Verwendung abgestimmte Wärmeverteilung über den Bestrahlungsbereich ist so begünstigt.

In dem gezeigten Beispiel ermöglicht die Anordnung der Wärmequellen 150, 152 in den Seitengehäusen 114, 116 zudem eine Abschattung des Bestrahlungsbereichs vor direkter Infrarotstrahlung der Wärmequellen 150, 152 mittels der Seitengehäuse 114, 116. Der Anteil umgelenkter anstatt direkter Infrarotstrahlung für eine Wärmebehandlung lässt sich so weiter erhöhen bzw. durch geeignete Wahl der Seitengehäuse 114, 116 einstellen.

Die Wärmequellen 150, 152 sind zu verschiedenen Seiten des Reflektors 140 untergebracht. Um dabei die Infrarotstrahlung jeder der Wärmequellen 150, 152 in die Bestrahlungsrichtung B umzulenken, weist der Reflektor mehrere Reflektionsflächen 142, 144 auf, die verschieden ausgerichtet sind. Die Reflektionsflächen 142, 144 erstrecken sich jeweils angrenzend an einen Mittenbereich MR des Reflektors 140. Die Reflektionsflächen 142, 144 sind so angeordnet, dass wenigstens ein Teil der Infrarotstrahlung, die von jedweder der Wärmequellen 150, 152 abgestrahlt wird, auf jeweils wenigstens eine der Reflektionsflächen 142, 144, die der jeweiligen Wärmequelle 150, 152 zugewandt angeordnet ist, trifft und in die Bestrahlungsrichtung B umgelenkt wird. Dabei sind die Reflektionsflächen 142, 144 beispielsweise dachförmig bzw. in Form eines umgedrehten 'V' angeordnet. Infrarotstrahlung, die von den Wärmequellen 150, 152 seitlich auf jeweils eine der Reflektionsflächen 142, 144 trifft, wird so mittels der Reflektionsflächen 142, 144 in die Bestrahlungsrichtung B umgelenkt.

Die Anordnung der Wärmequellen 150, 152 zu verschiedenen Seiten des Reflektors 140 begünstigt einen breiten Bestrahlungsbereich bei zugleich kompakter, insbesondere flacher, Ausgestaltung der Infrarotstrahler-Anordnung 100. Die Anordnung der Wärmequellen 150, 152 zu verschiedenen Seiten des Reflektors 140 begünstigt dabei außerdem eine gleichmäßige, insbesondere symmetrische, Wärmeverteilung über die Breite des Bestrahlungsbereichs. Das ist vorteilhaft, beispielsweise bei typischen Wärmebehandlungen im Bereich der Wirbelsäule.

Die im Beispiel von Fig. 1 gezeigte längliche Erstreckung der Wärmequellen 150, 152, des Reflektors 140 und der Öffnung 112 begünstigt außerdem einen entsprechend langgestreckten Behandlungsbereich mit gleichmäßiger Wärmeverteilung über die Länge des Behandlungsbereichs. Das ist vorteilhaft, beispielsweise für Wärmebehandlungen entlang einem längeren Wirbelsäulenabschnitt.

Die Infrarotstrahler-Anordnung 100 begünstigt so zudem eine verhältnismäßig gleichmäßige Leistungsdichte der Infrarotstrahlung in einem Volumen oberhalb eines zu behandelnden Hautbereichs, beispielsweise einem Volumen, das sich angrenzend an den Hautbereich von einer tiefsten Stelle in Strahlungsrichtung gemessen um etwa 10 cm entgegen der Bestrahlungsrichtung erstreckt. Selbst bei Unebenheiten des bestrahlten Hautbereichs, wie sie beispielsweise durch die Wirbelsäulenkrümmungen auftreten, ist so ein gleichmäßiger Wärmeeintrag in dem gesamten Hautbereich möglich.

In dem Beispiel von Fig. 1 umfasst die Infrarotstrahler-Anordnung 100 zudem Wärmesensoren 160, 162. Diese sind entlang dem Mittenbereich MR des Reflektors 140 zwischen den Reflektionsflächen 142, 144 angeordnet. Der Reflektor 140 weist dazu Reflektoraussparungen 146, 148 auf, unter denen jeweils auf einem Boden 110 des Gestells 105 eine Wärmesensoraufnahme 120, 122 angeordnet ist. Das gestattet eine Anordnung der Wärmesensoren 160, 162 hinter den Reflektionsflächen 142, 144. Auf diese Weise sind die Wärmesensoren 160, 162 vor direkter Infrarotstrahlung, die von den Wärmequellen 150, 152 abgestrahlt wird und die sonst ein Messergebnis der Wärmesensoren 160, 162 verfälschen könnte, abgeschirmt. Die Wärmesensoren 160, 162 sind bevorzugt so ausgerichtet, dass ein Messbereich jedes der Wärmesensoren 160, 162 mit dem Bestrahlungsbereich der Infrarotstrahler-Anordnung 100 wenigstens teilweise überlappt.

Mittels der Wärmesensoren 160, 162 lässt sich eine Oberflächentemperatur des Hautbereichs während des Aufbringens von Infrarotstrahlung in dem Hautbereich erfassen. Eine Wirkung der Infrarotstrahlung auf die Oberflächentemperatur in dem bestrahlten Hautbereich lässt sich so, beispielsweise mithilfe einer Steuerungseinrichtung, wie nachfolgend beschrieben, etwa kontinuierlich oder in beliebigen zeitlichen Abständen, sensorisch überwachen.

In einigen Beispielen der Infrarotstrahler-Anordnung 100 sind die Wärmequellen 150, 152 als steuerbare Wärmequellen, beispielsweise steuerbare Halogenröhren, ausgebildet. In Verbindung mit einer sensorischen Erfassung der Oberflächentemperatur in dem behandelten Hautbereich lässt sich die Heizleistung der Wärmequellen 150, 152, beispielsweise mithilfe einer Steuerungseinrichtung, in einigen Beispielen automatisch regeln.

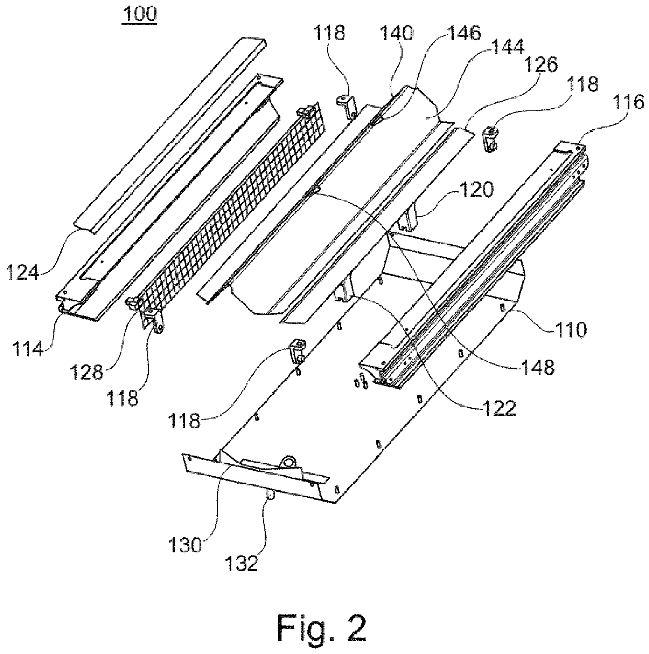

Fig. 2 zeigt schematisch eine Explosionsansicht der Infrarotstrahler-Anordnung 100. Gleiche Bezugszeichen wie in Fig. 1 bezeichnen dabei gleiche Merkmale.

Wie in Fig. 2 erkennbar, ist der Schweißauffang 130 schalenförmig im unteren Bereich des Bodens 110 ausgebildet. Über einen Schweißauslass 132 lässt sich aufgefangener Schweiß aus dem Schweißauffang 130 abführen.

Fig. 2 zeigt ferner exemplarisch ein Schutzgitter 128. Ein solches Schutzgitter 128 kann je nach Erfordernis in einigen Beispielen vor jeder der Wärmequellen 150, 152 zum besseren Schutz vor unbeabsichtigter Berührung der Wärmequellen 150, 152 durch einen unachtsamen Benutzer vorgesehen sein.

Die Schweißabtropfkonturen 124, 126 sind jeweils als separate Teile ausgebildet, die an den Seitengehäusen 114, 116 angebracht sind. Die Schweißabtropfkonturen 124, 126 dienen dabei in einigen Beispielen zugleich als Federelemente, die bei einem Anbringen der Infrarotstrahler-Anordnung 100 an einer Benutzeraufnahme das Gehäuse 105 gegenüber der Benutzeraufnahme federnd abstützen.

Erkennbar ist in Fig. 2 zudem der als separates Teil, beispielsweise als Reflektorblech und/oder aus Metall, sowie reversibel vom Gehäuse 105 lösbar ausgebildete Reflektor 140.

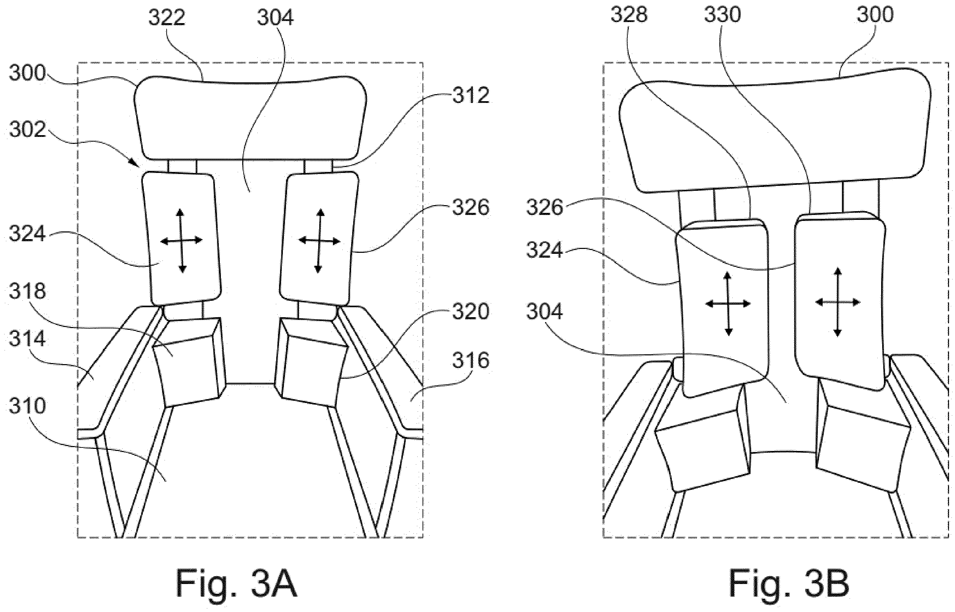

Fig. 3A und 3B zeigen schematisch und exemplarisch eine Benutzeraufnahme 300. Die Benutzeraufnahme 300 ist zur Verwendung in einem Wärmebehandlungssystem vorgesehen, beispielsweise in Verbindung mit der Infrarotstrahler-Anordnung 100 wie voranstehend im Zusammenhang mit Fig. 1 und 2 beschrieben. In dem gezeigten Beispiel ist die Benutzeraufnahme 300 zur sitzenden Aufnahme eines Benutzers vorgesehen. Die Benutzeraufnahme 300 umfasst zu diesem Zweck eine Stützstruktur in Form eines Sitzes.

Die Benutzeraufnahme 300 weist eine dem Benutzer zugewandte Anliegefläche 302 auf. Darin ist eine Aussparung 304 gemäß einer zur Wärmebehandlung vorgesehenen Körperregion des Benutzers vorgesehen. Die Benutzeraufnahme 300 umfasst zudem eine Sitzfläche 310, eine Rückenlehne 312 und Armlehnen 314, 316. Die Aussparung 304 ist im Bereich der Rückenlehne 312 angeordnet. Die Aussparung 304 ist dabei seitlich und nach oben durch Lendenstützen 318, 320, eine Nackenstütze 322 sowie durch der Höhe nach dazwischen angeordnete verstellbare Anliegeelemente 324, 326 begrenzt.

Die Benutzeraufnahme 300 ist dazu vorgesehen, dass eine Infrarotstrahler-Anordnung, beispielsweise die Infrarotstrahler-Anordnung 100, in einem von der Anliegefläche 302 abgewandten Bereich der Benutzeraufnahme 300 angebracht wird. Eine Bestrahlung mit Infrarotstrahlung mittels der Infrarotstrahler-Anordnung erfolgt dabei durch die Aussparung 304.

Die verstellbaren Anliegeelemente 324, 326 gestatten dabei, die Anliegefläche 302 an die Körperform, beispielsweise die Körpergröße, des jeweiligen Benutzers anzupassen. Dadurch ist eine geeignete Stützung des Körpers des Benutzers während einer Wärmebehandlung erzielbar. In dem gezeigten Beispiel lässt sich so beispielsweise mittels der verstellbaren Anliegelemente 324, 326 eine geeignete Stützung der Kyphose des Benutzers erzielen.

Ein Anpassen der Anliegefläche 302 an die Körperform, insbesondere die Körpergröße, mittels der verstellbaren Anliegeelemente 324, 326 gestattet außerdem, ein Verletzungsrisiko für den Benutzer zu verringern. Ein solches Verletzungsrisiko kann sich insbesondere bei Benutzern von verhältnismäßig geringer Körpergröße, beispielsweise bei Kindern oder jungen Jugendlichen, infolge eines möglichen Durchrutschens von Körperteilen des Benutzers durch die Aussparung 304 auf erhitzte Bereiche der Infrarotstrahler-Anordnung 100 ergeben.

Die verstellbaren Anliegeelemente 324, 326 gestatten in dem gezeigten Beispiel außerdem, den Bestrahlungsbereich an ein jeweiliges Erfordernis, beispielsweise gemäß der Körpergröße und/oder eines zur Behandlung vorgesehenen Körperbereichs, anzupassen. Dabei findet eine Begrenzung des Bestrahlungsbereichs mittels der verstellbaren Anliegeelemente 324, 326 durch Abschatten von nicht zur Behandlung vorgesehenen Hautpartien statt. Zu diesem Zweck weisen die verstellbaren Anliegeelemente 324, 326 an ihrer Rückseite, d.h. an der der Anliegefläche 302 abgewandten Seite, Blendenelemente 328, 330, beispielsweise aus Metall, auf.

In anderen Beispielen von Benutzeraufnahmen der vorgestellten Art sind zusätzlich oder alternativ verstellbare Blendenelemente getrennt von verstellbaren Anliegeelementen ausgebildet. Dadurch ist ermöglicht, ein Anpassen des Bestrahlungsbereichs mittels der verstellbaren Blendenelemente wenigstens teilweise unabhängig von einem Anpassen der Anliegefläche an die Körperform des Benutzers, das mittels der verstellbaren Anliegeelemente erfolgen kann, vorzunehmen.

Um ein Verstellen der Anliegeelemente 324, 326 und/oder der Blendenelemente 328, 330 zu ermöglichen, sind diese in einigen Beispielen jeweils mittels wenigstens einer lösbaren Verbindung an der Stützstruktur der Benutzeraufnahme 300 angebracht. Eine lösbare Verbindung umfasst dabei beispielsweise eine Schraubverbindung, eine Bolzenverbindung, eine Klemmverbindungen, eine Magnetverbindung und/oder eine Bajonettverbindung.

Das Verstellen der Anliegeelemente 324, 326 und der Blendenelemente 328, 330 erfolgt in dem gezeigten Beispiel durch Verändern einer Position der jeweils wenigstens einen lösbaren Verbindung. In anderen Beispielen erfolgt das Verstellen der Anliegeelemente und/oder der Blendenelemente zusätzlich oder alternativ durch geeignetes Auswählen der Anliegeelemente und/oder der Blendenelemente aus einer Vielzahl verschieden ausgebildeter Anliegeelemente und/oder Blendenelemente sowie durch lösbares Verbinden der ausgewählten Anliegeelemente und/oder Blendenelemente mit der Stützstruktur der Benutzeraufnahme 300.

Wie in Fig. 3A und 3B durch die gekreuzten Doppelpfeile angedeutet, ist jedes der verstellbaren Anliegeelemente 324, 326 sowohl in vertikaler Richtung als auch in lateraler Richtung verstellbar. Fig. 3A zeigt dabei die verstellbaren Anliegeelemente 324, 326 auf einer mittleren Höhe bei breitestmöglicher Anliegefläche 302 im Bereich der Kyphose. Das entspricht zugleich einem breitestmöglichen Bestrahlungsbereich durch die Aussparung 304.

Fig. 3B zeigt dagegen die verstellbaren Anliegeelemente 324, 326 ebenfalls auf einer mittleren Höhe, aber bei schmalster Anliegefläche 302. Das entspricht zugleich einem schmalsten Bestrahlungsbereich durch die Aussparung 304.

Das in Fig. 3A und 3B gezeigte Beispiel der Benutzeraufnahme 300 ist zur sitzenden Aufnahme eines Benutzers vorgesehen. Die Aussparung 304 ist dabei entsprechend einem Rückenbereich des Benutzers angeordnet. Andere Beispiele von Benutzeraufnahmen der vorgestellten Art sind davon abweichend oder zusätzlich zur stehenden und/oder liegenden Aufnahme eines Benutzers vorgesehen. Zudem ist in anderen Beispielen von Benutzeraufnahmen der vorgestellten Art wenigstens eine Aussparung entsprechend einem anderen Körperbereich als einem Rückenbereich des Benutzers angeordnet.

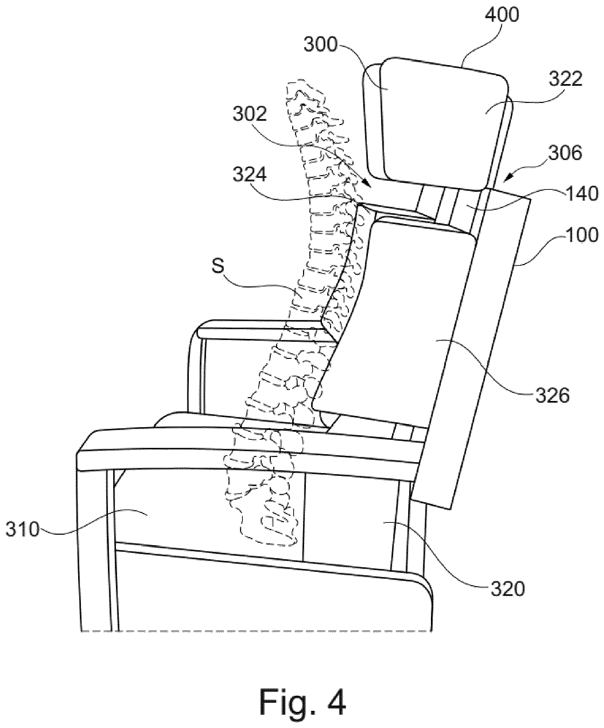

Fig. 4 zeigt schematisch und exemplarisch ein Wärmebehandlungssystem 400. Das Wärmebehandlungssystem 400 umfasst eine Benutzeraufnahme 300 wie im Zusammenhang mit Fig. 3A und 3B beschrieben und eine Infrarotstrahler-Anordnung 100 wie im Zusammenhang mit Fig. 1 und 2 beschrieben. Gleiche Bezugszeichen wie in Fig. 1 bis 3B bezeichnen darin gleiche Merkmale.

Die Infrarotstrahler-Anordnung 100 ist in einem von der Anliegefläche 302 abgewandten Bereich 306 der Benutzeraufnahme 300 an einer Rückseite der Rückenlehne 312 angeordnet. Die Infrarotstrahler-Anordnung 100 ist dabei derart angeordnet, dass die Bestrahlungsrichtung der Infrarotstrahler-Anordnung 100 durch die Aussparung 304 der Benutzeraufnahme 300 in Richtung auf den Benutzer gerichtet ist.

Aus der Position der Infrarotstrahler-Anordnung 100 und der geneigten Rückenlehne 312 ist außerdem erkennbar, dass die Infrarotstrahler-Anordnung 100 dabei Schweißabsonderungen des Benutzers verstärkt ausgesetzt ist. Die Ausrichtung der Infrarotstrahler-Anordnung 100 bewirkt dabei ein Abfließen von auftreffendem Schweiß in Richtung auf den Schweißauffang 130 im unteren Bereich des Gestells 105 der Infrarotstrahler-Anordnung 100.

In Fig. 4 ist exemplarisch die Wirbelsäule S eines Benutzers dargestellt. Dabei ist erkennbar, dass eine Stützung der Wirbelsäule S sowohl durch die Lendenstützen 318, 320 als auch durch die verstellbaren Anliegeelemente 324, 326 erfolgt. Zudem ist erkennbar, dass ein Abstand zwischen der Infrarotstrahler-Anordnung 100 und einem zu bestrahlenden Hautbereich infolge der Krümmungen der Wirbelsäule S variiert. Wie im Zusammenhang mit Fig. 1 beschrieben, begünstigt die Infrarotstrahler-Anordnung 100 dabei jedoch einen gleichmäßigen Wärmeeintrag in verschiedene Bereiche des Bestrahlungsbereichs.

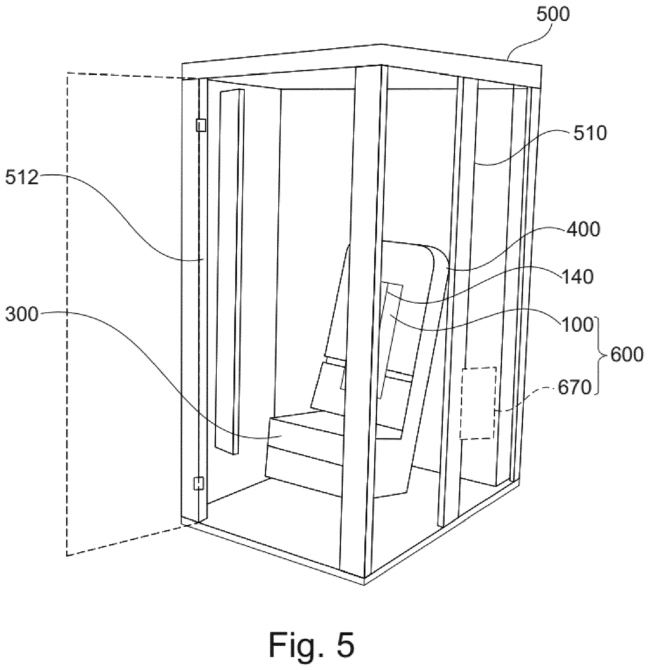

Fig. 5 zeigt schematisch und exemplarisch eine Wärmekabine 500. Die Wärmekabine 500 umfasst eine Kabinenwand 510 und eine Kabinentür 512, die zusammen einen Innenraum der Wärmekabine 500 begehbar umgeben. Die Wärmekabine 500 umfasst außerdem ein Wärmebehandlungssystem 400, das in dem Innenraum der Wärmekabine 500 angeordnet ist.

Bei dem Wärmebehandlungssystem 400 handelt es sich um ein Wärmebehandlungssystem wie voranstehend im Zusammenhang mit Fig. 4 beschrieben. Zur Benutzung des Wärmebehandlungssystems 400 betritt ein Benutzer den Innenraum der Wärmekabine 500 durch die verschließbare Kabinentür 512. Gegenüber einem freistehenden Wärmebehandlungssystem begünstigt die Wärmekabine 500 bei Bedarf eine Durchwärmung des gesamten Körpers des Benutzers.

In einigen Beispielen ist die Wärmekabine 500 als Druckkammer zum Zweck hyperbarer Therapieanwendungen ausgebildet. Die Wärmekabine 500 ist dabei außerdem dazu vorgesehen, Wärmebehandlungen mittels des Wärmebehandlungssystems 400 gleichzeitig mit hyperbaren Therapieanwendungen zu ermöglichen.

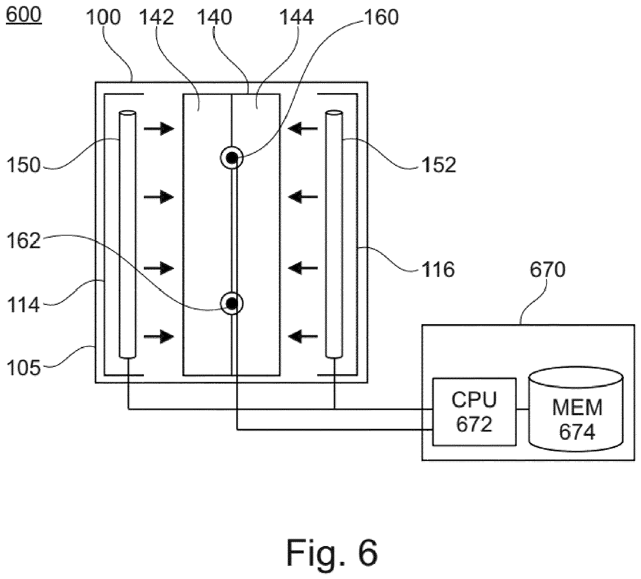

In einigen Beispielen umfasst das Wärmebehandlungssystem 400 zudem eine Steuerungseinrichtung 670, wie in Fig. 5 gestrichelt dargestellt und wie nachstehend im Zusammenhang mit Fig. 6 näher beschrieben. In einigen solcher Beispiele stellen die Steuerungseinrichtung 670 und die Infrarotstrahler-Anordnung 100 zusammenwirkende Teile einer Wärmebehandlungsvorrichtung 600 des Wärmebehandlungssystems 400 dar.

Fig. 6 zeigt schematisch und exemplarisch eine Wärmebehandlungsvorrichtung 600. Die Wärmebehandlungsvorrichtung 600 umfasst eine Infrarotstrahler-Anordnung 100 wie im Zusammenhang mit Fig. 1 und 2 beschrieben und eine Steuerungseinrichtung 670 mit einer Prozessoreinheit 672 und einer Speichervorrichtung 674. Die Steuerungseinrichtung 670 ist mit den Wärmesensoren 160, 162 und mit den Wärmequellen 150, 152, bei denen es sich in diesem Fall um steuerbare Wärmequellen handelt, operativ verbunden.

Die Steuerungseinrichtung 670 ist dazu ausgebildet, eine Heizleistung jeder der Wärmequellen 150, 152 zu steuern. Dazu ist die Steuerungseinrichtung 670 mit den Wärmequellen 150, 152 operativ verbunden. Die Verbindung erfolgt beispielsweise über einen Dimmer (nicht dargestellt), der mittels der Steuerungseinrichtung 670 steuerbar ist. In anderen Beispielen ist die Steuerungseinrichtung 670 dazu ausgebildet, an jede der Wärmequellen 150, 152 einen gemäß einer vorgesehenen Heizleistung bemessenen Heizstrom auszugeben.

Die Steuerungseinrichtung 670 ist ferner dazu ausgebildet, von jedem der Wärmesensoren 160, 162 Sensorsignale zu empfangen, die auf eine mittels des jeweiligen Wärmesensors 160, 162 erfasste Oberflächentemperatur hinweisen. Ein Sensorsignal von jedwedem der Wärmesensoren 160, 162 weist dabei auf eine Temperatur in dem Erfassungsbereich des jeweiligen Wärmesensors 160, 162 hin.

Die Steuerungseinrichtung 670 ist dazu ausgebildet, eine Heizleistung jeder der Wärmequellen 150, 152 wenigstens teilweise auf der Grundlage einer mittels der Wärmesensoren 160, 162 erfassten Oberflächentemperatur in einem Hautbereich des Benutzers, der sich im Bestrahlungsbereich der Wärmebehandlungsvorrichtung 600 befindet, zu steuern, insbesondere temperaturabhängig zu regeln. Dafür ist die Prozessoreinheit 672 dazu programmiert, ein oder mehrere Sensorsignale, die mittels eines oder mehrerer der Wärmesensoren 160, 162 erzeugt und an die Steuerungseinrichtung 670 ausgegeben und von der Steuerungseinrichtung 670 empfangen werden, mit wenigstens einer Bezugstemperatur zu vergleichen und in Abhängigkeit von dem Vergleichsergebnis die Heizleistung wenigstens einer der Wärmequellen 150, 152 zu verändern. Ein Verändern der Heizleistung kann dabei einem Verringern oder einem Erhöhen der Heizleistung entsprechen.

In einigen Beispielen der Wärmebehandlungsvorrichtung 600 ist eine Funktionalität der Prozessoreinheit 672 durch Programmcode bestimmt, der mittels der Speichervorrichtung 674 gespeichert ist und von der Prozessoreinheit 672 ausgeführt wird. In einigen Beispielen erfolgt zudem eine Heizleistungsregelung mittels der Steuerungseinrichtung 670 durch Vergleichen einer erfassten Temperatur mit einer oder mehreren Bezugstemperaturen, die mittels der Speichervorrichtung 674 gespeichert sind.

### Bezugszeichenliste

- 100: Infrarotstrahler-Anordnung
- 105: Gestell
- 110: Boden
- 112: Öffnung
- 114, 116: Seitengehäuse
- 118: Befestigungsmittel
- 120, 122: Wärmesensoraufnahme
- 124, 126: Schweißabtropfkontur
- 128: Schutzgitter
- 130: Schweißauffang
- 132: Schweißauslass
- 140: Reflektor
- 142, 144: Reflektionsfläche
- 146, 148: Reflektoraussparung
- 150, 152: Wärmequelle
- 300: Benutzeraufnahme
- 302: Anliegefläche
- 304: Aussparung
- 306: abgewandter Bereich
- 310: Sitzfläche
- 312: Rückenlehne
- 314, 316: Armlehne
- 318, 320: Lendenstütze
- 322: Nackenstütze
- 324, 326: verstellbares Anliegeelement
- 328, 330: Blendenelement
- 400: Wärmebehandlungssystem
- 500: Wärmekabine
- 510: Kabinenwand
- 512: Kabinentür
- 600: Wärmebehandlungsvorrichtung
- 670: Steuerungseinrichtung
- 672: Prozessoreinheit
- 674: Speichervorrichtung
- B: Bestrahlungsrichtung
- MR: Mittenbereich
- S: Wirbelsäule

## Patentansprüche

1. Infrarotstrahler-Anordnung (100) für ein Wärmebehandlungssystem (400) umfassend:
wenigstens eine Wärmequelle (150, 152),
wenigstens einen Reflektor (140, 142, 144), der dazu ausgebildet ist, Infrarotstrahlung, die mittels der wenigstens einen Wärmequelle (150, 152) erzeugt ist, in Richtung auf einen Benutzer des Wärmebehandlungssystems (400) umzulenken, und
wenigstens ein Gestell (105), an dem die wenigstens eine Wärmequelle (150, 152) und der wenigstens eine Reflektor (140, 142, 144) angebracht sind,
wobei:
während einer vorgesehenen Verwendung des Wärmebehandlungssystems (400) der wenigstens eine Reflektor (140, 142, 144) mechanischer und/oder chemischer Wechselwirkung mit einer Umgebung des Wärmebehandlungssystems (400) ausgesetzt ist, und
der wenigstens eine Reflektor (140, 142, 144) reversibel lösbar an dem wenigstens einen Gestell (105) angebracht ist.

2. Infrarotstrahler-Anordnung nach Anspruch 1, wobei die Infrarotstrahler-Anordnung (100) so ausgebildet ist, dass der wenigstens eine Reflektor (140, 142, 144) von dem wenigstens einen Gestell (105) reversibel lösbar ist, wenn die Infrarotstrahler-Anordnung (100) an einer Benutzeraufnahme (300) des Wärmebehandlungssystems (400) angebracht ist.

3. Infrarotstrahler-Anordnung nach Anspruch 1 oder Anspruch 2, wobei das wenigstens eine Gestell (105) ausgebildet ist zum Anbringen der Infrarotstrahler-Anordnung (100) an einer Benutzeraufnahme (300) des Wärmebehandlungssystems (400).

4. Infrarotstrahler-Anordnung nach einem der vorhergehenden Ansprüche, wobei die Infrarotstrahler-Anordnung (100) wenigstens zwei Wärmequellen (150, 152) umfasst, die zu verschiedenen Seiten eines Mittenbereichs (MR) des wenigstens einen Reflektors (140, 142, 144) angeordnet sind.

5. Infrarotstrahler-Anordnung nach Anspruch 4, wobei der wenigstens eine Reflektor (140, 142, 144) mehrere Reflektionsflächen (142, 144) umfasst, die zu verschiedenen Seiten des Mittenbereichs (MR) des Reflektors (140, 142, 144) ausgebildet sind, wobei jeder der wenigstens zwei Wärmequellen (150, 152) jeweils wenigstens einer der Reflektionsflächen (142, 144) zugeordnet ist.

6. Infrarotstrahler-Anordnung nach einem der vorhergehenden Ansprüche, ferner umfassend wenigstens eine Wärmesensoraufnahme (120, 122), die dazu vorgesehen ist, wenigstens einen Wärmesensor (160, 162) des Wärmebehandlungssystems (400) aufzunehmen, der dazu ausgebildet ist, während der vorgesehenen Verwendung des Wärmebehandlungssystems (400) eine Temperatur in einem Hautbereich des Benutzers zu erfassen, wobei der wenigstens eine Reflektor (140, 142, 144) dazu ausgebildet ist, die Infrarotstrahlung wenigstens teilweise in Richtung auf den Hautbereich des Benutzers umzulenken, insbesondere wobei die Wärmesensoraufnahme (120, 122) dazu ausgebildet ist, den wenigstens einen Wärmesensor (160, 162) an einer in Bezug auf die wenigstens eine Wärmequelle (150, 152) abgewandten Seite des wenigstens einen Reflektors (140, 142, 144) aufzunehmen.

7. Infrarotstrahler-Anordnung nach Anspruch 6, wobei der wenigstens eine Reflektor (140, 142, 144) wenigstens eine Reflektoraussparung (146, 148) aufweist und die wenigstens eine Wärmesensoraufnahme (120, 122) dazu ausgebildet ist, den wenigstens einen Wärmesensor (160, 162) derart aufzunehmen, dass ein Messfeld des wenigstens einen Wärmesensors (160, 162) sich wenigstens teilweise durch die wenigstens eine Reflektoraussparung (146, 148) erstreckt.

8. Infrarotstrahler-Anordnung nach einem der vorhergehenden Ansprüche, wobei der wenigstens eine Reflektor (140, 142, 144) ein Reflektorblech umfasst.

9. Infrarotstrahler-Anordnung nach einem der vorhergehenden Ansprüche, wobei die Infrarotstrahler-Anordnung (100) ferner wenigstens eine Schweißabtropfkontur (124, 126) und/oder wenigstens einen Schweißauffang (130, 132) umfasst, und wobei während der vorgesehenen Verwendung des Wärmebehandlungssystems (400) der wenigstens eine Reflektor (140, 142, 144) einer Schweißabsonderung des Benutzers ausgesetzt ist.

10. Wärmebehandlungssystem (400) umfassend:
eine Infrarotstrahler-Anordnung (100) nach einem der vorhergehenden Ansprüche, und
eine Benutzeraufnahme (300), an der die Infrarotstrahler-Anordnung (100) angebracht ist und die zur sitzenden, liegenden und/oder stehenden Aufnahme des Benutzers des Wärmebehandlungssystems (400) ausgebildet ist.

11. Wärmebehandlungssystem nach Anspruch 10, wobei:
die Benutzeraufnahme (300) ferner wenigstens eine Anliegefläche (302) umfasst, die dem Benutzer zugewandt ist und die eine Aussparung (304) aufweist, die mit einem zur Wärmebehandlung vorgesehenen Hautbereich des Benutzers in einer Bestrahlungsrichtung wenigstens teilweise überlappt, und
die Infrarotstrahler-Anordnung (100) in einem von der Anliegefläche (302) abgewandten Bereich (306) der Benutzeraufnahme (300) angeordnet ist derart, dass Infrarotstrahlung, die mittels der wenigstens einen Wärmequelle (150, 152) erzeugt ist, mittels des wenigstens einen Reflektors (140, 142, 144) durch die Aussparung (304) in Richtung auf den Benutzer umgelenkt ist.

12. Wärmebehandlungssystem nach Anspruch 11, wobei:
die Benutzeraufnahme (300) eine Stützstruktur in Form eines Sitzes, einer Liege und/oder einer Lehne umfasst und zur sitzenden, liegenden und/oder stehenden Aufnahme des Benutzers vorgesehen ist, und
die Anliegefläche (302) einer dem Benutzer zugewandten Seite der Stützstruktur entspricht.

13. Wärmebehandlungssystem nach Anspruch 11 oder Anspruch 12, wobei die wenigstens eine Wärmequelle (150, 152) derart angeordnet ist, dass ein überwiegender Anteil der Infrarotstrahlung, die mittels der wenigstens einen Wärmequelle (150, 152) erzeugt ist und durch die Aussparung (304) in Richtung auf den Benutzer tritt, umgelenkte Infrarotstrahlung ist.

14. Wärmebehandlungssystem nach einem der Ansprüche 10 bis 13, wobei die wenigstens eine Wärmequelle (150, 152) eine steuerbare Wärmequelle ist, und wobei das Wärmebehandlungssystem (400) ferner umfasst:
wenigstens einen Wärmesensor (160, 162), der in der Infrarotstrahler-Anordnung (100) aufgenommen ist und der dazu ausgebildet ist, während der vorgesehenen Verwendung des Wärmebehandlungssystems (400) eine Temperatur in einem Hautbereich des Benutzers zu erfassen und ein Sensorsignal auszugeben, das auf die erfasste Temperatur hinweist, und
eine Steuerungseinrichtung (670), die dazu ausgebildet ist, das Sensorsignal zu empfangen und wenigstens teilweise auf der Grundlage des empfangenen Sensorsignals die wenigstens eine Wärmequelle (150, 152) in Bezug auf eine Heizleistung zu steuern,
wobei die wenigstens eine Wärmequelle (150, 152), der wenigstens eine Reflektor (140, 142, 144), der wenigstens eine Wärmesensor (160, 162) und die Steuerungseinrichtung (670) Bestandteile einer Wärmebehandlungsvorrichtung (600) des Wärmebehandlungssystems (400) sind.

15. Wärmekabine (500) umfassend ein Wärmebehandlungssystem nach einem der Ansprüche 10 bis 14.
